(19) Europäisches Patentamt
European Patent Office
Office européen des brevets

(11) EP 0 755 185 B1

(12) # EUROPÄISCHE PATENTSCHRIFT

(45) Veröffentlichungstag und Bekanntmachung des Hinweises auf die Patenterteilung:
**13.12.2000 Patentblatt 2000/50**

(21) Anmeldenummer: **95914324.9**

(22) Anmeldetag: **29.03.1995**

(51) Int. Cl.$^7$: **A01N 43/10**, C07D 333/38

(86) Internationale Anmeldenummer:
**PCT/EP95/01173**

(87) Internationale Veröffentlichungsnummer:
**WO 95/27397 (19.10.1995 Gazette 1995/45)**

(54) **Verwendung von Dibrom-thiophen-carbonsäure-Derivaten als Mikrobizide**
Use of Dibromo-thiophen-carboxylic acid derivatives as microbicides
Utilisation de dérivés d'acide dibromothiophenecarboxylique comme microbicides

(84) Benannte Vertragsstaaten:
**BE CH DE ES FR GB GR IT LI NL**

(30) Priorität: **11.04.1994 DE 4412333**

(43) Veröffentlichungstag der Anmeldung:
**29.01.1997 Patentblatt 1997/05**

(73) Patentinhaber: **BAYER AG**
**51368 Leverkusen (DE)**

(72) Erfinder:
- **ELBE, Hans-Ludwig**
  **D-42329 Wuppertal (DE)**
- **ASSMANN, Lutz**
  **D-23701 Eutin (DE)**
- **TIEMANN, Ralf**
  **D-51375 Leverkusen (DE)**
- **ECKER, Uta**
  **D-42799 Leichlingen (DE)**
- **HÄNSSLER, Gerd**
  **D-51381 Leverkusen (DE)**
- **DEHNE, Heinz-Wilhelm**
  **D-53125 Bonn (DE)**

(56) Entgegenhaltungen:

| | |
|---|---|
| **EP-A- 0 258 790** | **EP-A- 0 379 003** |
| **EP-A- 0 450 355** | **EP-A- 0 590 458** |
| **DE-A- 1 813 195** | **FR-A- 1 267 782** |
| **US-A- 3 243 342** | **US-A- 3 536 473** |
| **US-A- 3 887 354** | **US-A- 4 542 145** |

- **LIEBIGS ANNALEN DER CHEMIE, 1979 WEINHEIM, DE;, Seiten 2043-2056, K. BEELITZ ET AL. 'Thiophenthiocarbonsäure-S-ester, Synthone für Thienothiochromone und Thienoazathiochromone'**
- **JOURNAL OF THE CHEMICAL SOCIETY, PERKIN TRANSACTIONS 1, 1973 LETCHWORTH, UK;, Seiten 1766-1773, D.J.CHADWICK 'Esters of Furan-, Thiophen-, and N-Methylpyrrole- 2-carboxylic Acids. Bromination of Methyl Furan-2-carboxylate, Furan-2-carbaldehyde and Thiophen-2-carbaldehyde in the Presence of Aluminium Chloride'**
- **BERICHTE DER DEUTSCHEN CHEMISCHEN GESELLSCHAFT, Bd. 18, 1885 BERLIN,DE, Seite 2308 R.BONZ 'Über die Bromirung der alpha- und der beta-Thiophensäure'**
- **PATENT ABSTRACTS OF JAPAN vol. 11 no. 369 (C-461) [2816] ,2.Dezember 1987 & JP,A,62 138489 (TOKUYAMA SODA CO. ) 22.Juni 1987,**
- **JOURNAL OF MEDICINAL CHEMISTRY , Bd. 30, 1987 WASHINGTON, US;, Seiten 1036-1040, J.B. PRESS ET AL. 'Thromboxane Synthetase Inhibitors and Antihypertensive Agents. 3. N-[(1H-Imidazol-1-yl)alkyl]heteroaryl Amides as Potent Enzyme Inhibitors'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 755 185 B1

Printed by Xerox (UK) Business Services
2.16.7 (HRS)/3.6

## Beschreibung

Die vorliegende Erfindung betrifft die Verwendung von teilweise bekannten Dibrom-thiophen-carbonsäure-Derivaten zur Bekämpfung von unerwünschten Mikroorganismen. Außerdem betrifft die Erfindung auch neue Dibrom-thiophen-carbonsäure-Derivate und ein Verfahren zu deren Herstellung.

Es ist bereits bekannt, daß bestimmte Halogenthiophen-carbonsäureamid-Derivate zur Bekämpfung von Pflanzenkrankheiten eingesetzt werden können (vgl. EP-A 0 450 355). Die Wirksamkeit dieser vorbekannten Verbindungen ist jedoch insbesondere bei niedrigen Aufwandmengen nicht in allen Anwendungsgebieten völlig zufriedenstellend.

Aus der EP-A 0 590 458 geht hervor, daß bestimmte Dibrom-thiophencarbonsäure-amide fungizide Eigenschaften besitzen. Entsprechende Ester werden aber nicht erwähnt.

Gemäß US-A 3 243 342 lassen sich Halogen-thiophen-carbonsäure-anilide, wie 4,5-Dibrom-thiophencarbonsäure-(2,3-dichlor-phenyl)-amid, als Keimabtötungsmittel einsetzen. Ester dieses Typs werden jedoch nicht beschrieben.

In der DE-A 1 813 195 werden Trichlorthiophen-2-carbonsäureamide mit fungizider Wirksamkeit offenbart. Hinweise auf Dibrom-thiophen-Derivate sind allerdings nicht vorhanden.

Weiterhin sind bestimmte 4,5-Dibrom-thiophen-2-carbonsäure-Derivate bekannt, wie beispielsweise die Verbindungen 4,5-Dibrom-thiophen-2-carbonsäure-methylester (CA-Reg. No. 62224-24-2) und -ethylester (CA-Reg. No. 62224-25-3) und -1,1-dimethylethylester (CA-Reg. No. 62224-26-4) und -2-propinylester (CA-Reg. No. 116 041-66-8) und -1-methyl-2-propinylester (CA-Reg. No. 131 818-24-1) und -3-butinylester (CA-Reg. No. 131 818-21-8) und -N,N-dimethylamid (CA-Reg. No. 111 859-96-2) und -N-ethoxy-amid (CA-Reg. No. 131 818-25-2) sowie 4,5-Dibrom-thiophen-2-carbonsäure (CA-Reg. No. 6324-10-3) und -carbonsäureamid (Beilstein Reg. No. 129 357).

Ferner werden in den folgenden Druckschriften zahlreiche Thiophencarbonsäure-Derivate erwähnt : Liebigs Ann. Chem. 1979, 2043 - 2056 , US-A 3 536 473 , US-A 3 887 354, EP-A 0 258 790 , EP-A 0 379 003, FR-A 1 267 782 , J. Med. Chem. 1987, 30 , 1036 - 1040 , US-A 4 542 145 , JP-A 62 - 138 489 , J. Chem. Soc. Perkin Trans. I 1973, 1766 - 1773 und Ber. 18, 2308 - 2215 (1885).

Über eine fungizide Wirksamkeit dieser Verbindungen ist bisher nichts bekannt.

Es wurde nun gefunden, daß die teilweise bekannten Dibrom-thiophen-carbonsäure-Derivate der Formel

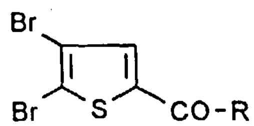

(I),

in welcher

R für die Gruppierung $-XR^1$ steht, worin

X für Sauerstoff oder Schwefel steht und

$R^1$ für Wasserstoff,
oder geradkettiges oder verzweigtes Alkyl mit 1 bis 12 Kohlenstoffatomen steht, das einfach bis fünffach, gleichartig oder verschieden substituiert sein kann durch Halogen, Cyano, Amino, Hydroxy, Mercapto, Carboxy, Nitro, jeweils geradkettiges oder verzweigtes Alkoxy, Alkylthio oder Alkoxycarbonyl mit jeweils 1 bis 6 Kohlenstoffatomen in den einzelnen Alkylteilen, sowie die Gruppierung $-NR^7R^8$, oder
für jeweils gegebenenfalls einfach bis fünffach, gleich oder verschieden durch Halogen substituiertes geradkettiges oder verzweigtes Alkenyl und Alkinyl mit 3 bis 6 Kohlenstoffatomen steht, oder
für Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im geradkettigen oder verzweigten, gegebenenfalls durch Cyano substituierten Alkylteil steht, wobei der Cycloalkylteil der beiden genannten Reste einfach bis fünffach, gleichartig oder verschieden substituiert sein kann durch geradkettiges oder verzweigtes Alkyl mit 1 bis 4 Kohlenstoffatomen, Halogen, geradkettiges oder verzweigtes Halogenalkyl mit 1 bis 4 Kohlenstoffatomen und 1 bis 5 gleichen oder verschiedenen Halogenatomen, Cyano, Alkylendioxo mit 1 bis 3 Kohlenstoffatomen, gegebenenfalls einfach bis dreifach, gleichartig oder verschieden durch Alkyl mit 1 bis 4 Kohlenstoffatomen substituiertes Cycloalkyl mit 3 bis 7 Kohlenstoffatomen und/oder durch Phenyl, welches seinerseits einfach bis dreifach, gleichartig oder verschieden substituiert sein kann durch geradkettiges

oder verzweigtes Alkyl mit 1 bis 4 Kohlenstoffatomen, Halogen und geradkettiges oder verzweigtes Halogenalkyl mit 1 bis 4 Kohlenstoffatomen und 1 bis 5 gleichen oder verschiedenen Halogenatomen, oder
für Tetrahydrofuran, Tetrahydrothiophen, Tetrahydropyran oder für Tetrahydrothiopyran steht, wobei jeder dieser Reste einfach bis dreifach, gleichartig oder verschieden substituiert sein kann durch geradkettiges oder verzweigtes Alkyl mit 1 bis 4 Kohlenstoffatomen, Halogen, geradkettiges oder verzweigtes Halogenalkyl mit 1 bis 4 Kohlenstoffatomen und 1 bis 5 gleichen oder verschiedenen Halogenatomen, Cyano, Alkylendioxo mit 1 bis 3 Kohlenstoffatomen, gegebenenfalls einfach bis dreifach, gleichartig oder verschieden durch Alkyl mit 1 bis 4 Kohlenstoffatomen substituiertes Cycloalkyl mit 3 bis 7 Kohlenstoffatomen und/oder durch Phenyl, welches seinerseits einfach bis dreifach, gleichartig oder verschieden substituiert sein kann durch geradkettiges oder verzweigtes Alkyl mit 1 bis 4 Kohlenstoffatomen, Halogen und geradkettiges oder verzweigtes Halogenalkyl mit 1 bis 4 Kohlenstoffatomen und 1 bis 5 gleichen oder verschiedenen Halogenatomen, oder
für Aryl mit 6 bis 10 Kohlenstoffatomen oder Aralkyl mit 6 bis 10 Kohlenstoffatomen im Arylteil und 1 bis 4 Kohlenstoffatomen im geradkettigen oder verzweigten, gegebenenfalls durch Cyano substituierten Alkylteil steht, wobei jeder der beiden Reste im Arylteil einfach bis dreifach, gleichartig oder verschieden substituiert sein kann durch Halogen, Hydroxy, Cyano, Nitro, jeweils geradkettiges oder verzweigtes Alkyl, Alkoxy oder Alkylthio mit jeweils 1 bis 4 Kohlenstoffatomen, Cycloalkyl mit 3 bis 7 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 5 gleichen oder verschiedenen Halogenatomen und/oder durch Phenyl, das seinerseits einfach bis dreifach, gleichartig oder verschieden substituiert sein kann durch Halogen und/oder Alkyl mit 1 bis 4 Kohlenstoffatomen, oder
für 5- oder 6-gliedriges Hetaryl oder für 5- oder 6-gliedriges Hetarylalkyl mit 1 bis 6 Kohlenstoffatomen im geradkettigen oder verzweigten, gegebenenfalls durch Cyano substituierten Alkylteil steht, wobei der Hetarylteil jeweils 1 bis 3 Heteroatome, wie Sauerstoff, Schwefel und/oder Stickstoff, enthält und einfach bis dreifach, gleichartig oder verschieden substituiert sein kann durch geradkettiges oder verzweigtes Alkyl mit 1 bis 6 Kohlenstoffatomen, Halogen, Cyano und/oder geradkettiges oder verzweigtes Halogenalkyl mit 1 bis 4 Kohlenstoffatomen und 1 bis 5 gleichen oder verschiedenen Halogenatomen,

$R^7$ für geradkettiges oder verzweigtes Alkyl mit 1 bis 6 Kohlenstoffatomen steht, das einfach bis fünffach, gleichartig oder verschieden substituiert sein kann durch Halogen, Cyano, Amino, Hydroxy, Mercapto, Nitro, Carboxy, jeweils geradkettiges oder verzweigtes Alkoxy und Alkoxycarbonyl mit jeweils 1 bis 6 Kohlenstoffatomen in den Alkoxyteilen, Alkylamino und Dialkylamino mit 1 bis 6 Kohlenstoffatomen in jedem geradkettigen oder verzweigten Alkylteil, oder
für jeweils gegebenenfalls durch 1 bis 5 gleiche oder verschiedene Halogenatome substituiertes geradkettiges oder verzweigtes Alkenyl oder Alkinyl mit jeweils 3 bis 6 Kohlenstoffatomen steht, oder für Benzyl steht, das im Phenylteil einfach bis dreifach, gleichartig oder verschieden substituiert sein kann durch Halogen, Nitro, Cyano, geradkettiges oder verzweigtes Alkyl mit 1 bis 6 Kohlenstoffatomen, sowie jeweils geradkettiges oder verzweigtes Halogenalkyl, Halogenalkoxy oder Halogenalkylthio mit jeweils 1 bis 4 Kohlenstoffatomen und 1 bis 5 gleichen oder verschiedenen Halogenatomen und

$R^8$ für Wasserstoff oder für geradkettiges oder verzweigtes Alkyl mit 1 bis 6 Kohlenstoffatomen steht, das einfach bis fünffach, gleichartig oder verschieden substituiert sein kann durch Halogen, Cyano, Amino, Hydroxy, Mercapto, Nitro, Carboxy, jeweils geradkettiges oder verzweigtes Alkoxy und Alkoxycarbonyl mit jeweils 1 bis 6 Kohlenstoffatomen in den Alkoxyteilen, Alkylamino und Dialkylamino mit 1 bis 6 Kohlenstoffatomen in jedem geradkettigen oder verzweigten Alkylteil, oder
für jeweils gegebenenfalls durch 1 bis 5 gleiche oder verschiedene Halogenatome substituiertes geradkettiges oder verzweigtes Alkenyl oder Alkinyl mit jeweils 3 bis 6 Kohlenstoffatomen steht, oder
für Benzyl steht, das im Phenylteil einfach bis dreifach, gleichartig oder verschieden substituiert sein kann durch Halogen, Nitro, Cyano, geradkettiges oder verzweigtes Alkyl mit 1 bis 6 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 5 gleichen oder verschiedenen Halogenatomen, oder

$R^7$ und $R^8$ außerdem gemeinsam für zweifach verknüpftes Alkandiyl mit 4 bis 6 $CH_2$-Gruppen stehen, wobei gegebenenfalls eine $CH_2$-Gruppe durch O oder S ersetzt ist,

sehr gut zur Bekämpfung von unerwünschten Mikroorganismen geeignet sind.

Die Verbindungen der Formel (I) können in Abhängigkeit von der Art der Substituenten als geometrische und/oder optische Isomere oder Isomerengemische unterschiedlicher Zusammensetzung vorliegen. Die Erfindung

betrifft sowohl die Verwendung der reinen Isomeren als auch der Isomerengemische.

$R^1$ steht bevorzugt für Wasserstoff oder geradkettiges oder verzweigtes Alkyl mit 1 bis 8 Kohlenstoffatomen, das einfach bis vierfach, gleichartig oder verschieden substituiert sein kann durch Fluor, Chlor, Cyano, Amino, Hydroxy, Mercapto, jeweils geradkettiges oder verzweigtes Alkoxy und Alkylthio mit jeweils 1 bis 6 Kohlenstoffatomen sowie die Gruppierung $-NR^7R^8$, oder
für jeweils gegebenenfalls einfach bis dreifach, gleich oder verschieden durch Fluor, Chlor und/oder Brom substituiertes geradkettiges oder verzweigtes Alkenyl oder Alkinyl mit 3 bis 6 Kohlenstoffatomen, oder
für Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im geradkettigen oder verzweigten, gegebenenfalls durch Cyano substituierten Alkylteil, wobei der Cycloalkylteil der beiden genannten Reste einfach bis vierfach, gleichartig oder verschieden substituiert sein kann durch geradkettiges oder verzweigtes Alkyl mit 1 bis 4 Kohlenstoffatomen, Halogen, geradkettiges oder verzweigtes Halogenalkyl mit 1 bis 4 Kohlenstoffatomen und 1 bis 3 gleichen oder verschiedenen Halogenatomen, Cyano, Alkylendioxo mit 1 oder 2 Kohlenstoffatomen, gegebenenfalls durch Methyl oder Ethyl substituiertes Cyclohexyl oder Cyclopentyl und/oder durch Phenyl, das seinerseits einfach oder zweifach, gleichartig oder verschieden substituiert sein kann durch Methyl, Ethyl, Fluor, Chlor und/oder Halogenalkyl mit 1 oder 2 Kohlenstoffatomen und 1 bis 3 gleichen oder verschiedenen Halogenatomen, oder
für Tetrahydrofuran, Tetrahydrothiophen, Tetrahydropyran oder für Tetrahydrothiopyran, wobei jeder dieser Reste einfach bis dreifach, gleichartig oder verschieden substituiert sein kann durch geradkettiges oder verzweigtes Alkyl mit 1 bis 4 Kohlenstoffatomen, Halogen, geradkettiges oder verzweigtes Halogenalkyl mit 1 bis 4 Kohlenstoffatomen und 1 bis 3 gleichen oder verschiedenen Halogenatomen, Cyano, Alkylendioxo mit 1 oder 2 Kohlenstoffatomen, gegebenenfalls durch Methyl oder Ethyl substituiertes Cyclohexyl oder Cyclopentyl und/oder durch Phenyl, das seinerseits einfach oder zweifach, gleichartig oder verschieden substituiert sein kann durch Methyl, Ethyl, Fluor, Chlor und/oder Halogenalkyl mit 1 oder 2 Kohlenstoffatomen und 1 bis 3 gleichen oder verschiedenen Halogenatomen, oder
für Phenyl, Naphthyl, Phenylalkyl oder Naphthylalkyl mit 1 bis 4 Kohlenstoffatomen im geradkettigen oder verzweigten, gegebenenfalls durch Cyano substituierten Alkylteil, wobei jeder der vier genannten Reste im Arylteil einfach bis dreifach, gleichartig oder verschieden substituiert sein kann durch Halogen, Hydroxy, Cyano, Nitro, jeweils geradkettiges oder verzweigtes Alkyl, Alkoxy oder Alkylthio mit jeweils 1 bis 4 Kohlenstoffatomen, Cycloalkyl mit 3 bis 7 Kohlenstoffatomen, jeweils geradkettiges oder verzweigtes Halogenalkyl, Halogenalkoxy oder Halogenalkylthio mit jeweils 1 bis 2 Kohlenstoffatomen und 1 bis 5 gleichen oder verschiedenen Halogenatomen und/oder gegebenenfalls einfach bis dreifach, gleich oder verschieden durch Halogen und/oder Alkyl mit 1 bis 2 Kohlenstoffatomen substituiertes Phenyl, oder
für 5- oder 6-gliedriges Hetaryl oder
für 5- oder 6-gliedriges Hetarylalkyl mit 1 bis 4 Kohlenstoffatomen im geradkettigen oder verzweigten, gegebenenfalls durch Cyano substituierten Alkylteil, wobei der Hetarylteil jeweils 1 bis 3 Heteroatome, wie Sauerstoff, Schwefel und/oder Stickstoff, enthält und einfach bis dreifach, gleichartig oder verschieden substituiert sein kann durch geradkettiges oder verzweigtes Alkyl mit 1 bis 4 Kohlenstoffatomen, Fluor, Chlor, Brom, Cyano und Halogenalkyl mit 1 bis 2 Kohlenstoffatomen und 1 bis 5 gleichen oder verschiedenen Halogenatomen.

$R^7$ steht bevorzugt für geradkettiges oder verzweigtes Alkyl mit 1 bis 6 Kohlenstoffatomen, das einfach bis vierfach, gleichartig oder verschieden substituiert sein kann durch Fluor, Chlor, Cyano, Amino, Hydroxy, Carboxy sowie jeweils geradkettiges oder verzweigtes Alkoxy und Alkoxycarbonyl mit jeweils 1 bis 4 Kohlenstoffatomen in den Alkoxyteilen, oder
für gegebenenfalls durch 1 bis 3 Halogenatome substituiertes geradkettiges oder verzweigtes Alkenyl mit 3 bis 6 Kohlenstoffatomen,
für geradkettiges oder verzweigtes Alkinyl mit 3 bis 6 Kohlenstoffatomen, oder -
für Benzyl, das im Phenylteil einfach bis dreifach, gleichartig oder verschieden substituiert sein kann durch Fluor, Chlor und/oder geradkettiges oder verzweigtes Alkyl mit 1 bis 4 Kohlenstoffatomen.

$R^8$ steht bevorzugt für Wasserstoff, oder
für geradkettiges oder verzweigtes Alkyl mit 1 bis 6 Kohlenstoffatomen, das einfach bis vierfach, gleichartig oder verschieden substituiert sein kann durch Fluor, Chlor, Cyano, Amino, Hydroxy, Carboxy sowie jeweils geradkettiges oder verzweigtes Alkoxy und Alkoxycarbonyl mit jeweils 1 bis 4 Kohlenstoffatomen in den Alkoxyteilen, oder
für gegebenenfalls durch 1 bis 3 Halogenatome substituiertes geradkettiges oder verzweigtes Alkenyl mit

3 bis 6 Kohlenstoffatomen,
für geradkettiges oder verzweigtes Alkinyl mit 3 bis 6 Kohlenstoffatomen, oder
für Benzyl, das im Phenylteil einfach bis dreifach, gleichartig oder verschieden substituiert sein kann durch Fluor, Chlor und/oder geradkettiges oder verzweigtes Alkyl mit 1 bis 4 Kohlenstoffatomen.

$R^7$ und $R^8$ stehen außerdem gemeinsam bevorzugt für zweifach verknüpftes Alkandiyl mit 4 bis 6 $CH_2$-Gruppen, wobei gegebenenfalls eine $CH_2$-Gruppe durch O oder S ersetzt ist.

$R^1$ steht besonders bevorzugt für Wasserstoff, oder
für Methyl, Ethyl, n- oder i-Propyl, n-, i-, s- oder t-Butyl, n- oder i-Pentyl, n- oder i-Hexyl, n- oder i-Heptyl und n- oder i-Octyl, wobei jeder dieser Reste einfach bis dreifach, gleichartig oder verschieden substituiert sein kann durch Fluor, Chlor, Cyano, Amino, Hydroxy, Mercapto, Methoxy, Ethoxy, n-oder i-Propoxy, n-, i-, s- oder t-Butoxy, Methylthio, Ethylthio, n- oder i-Propylthio oder die Gruppierung $-NR^7R^8$, oder
für jeweils gegebenenfalls einfach bis dreifach, gleich oder verschieden durch Fluor und/oder Chlor substituiertes Allyl, n- oder i-Butenyl, n- oder i-Pentenyl, n- oder i-Hexenyl, Propargyl, n- oder i-Butinyl, n- oder i-Pentinyl und n- oder i-Hexinyl, oder
für jeweils gegebenenfalls im Cycloalkylteil ein- bis vierfach, gleich oder verschieden durch Methyl, Ethyl, n- oder i-Propyl, Fluor, Chlor, Brom, Trifluormethyl, Difluormethyl, Chlordifluormethyl, Bromdifluormethyl, Chlormethyl, Dichlormethyl, Cyano, Methylendioxo, Ethylendioxo, Methylcyclopentyl, Methylcyclohexyl, Ethylcyclopentyl, Ethylcyclohexyl, Phenyl, Chlorphenyl, Dichlorphenyl, Fluorophenyl, Difluorphenyl, Chlorfluorphenyl, Methylphenyl, Dimethylphenyl, Chlormethylphenyl, Fluormethylphenyl, Trifluormethylphenyl, Chlor-trifluormethylphenyl oder Fluor-trifluormethyl substituiertes Cyclopropyl, Cyclopropylmethyl, Cyclopropylethyl, Cyclopropylpropyl, Cyclopropyl-1-cyanoeth-1-yl, Cyclopropyl-cyanomethyl, Cyclopentyl, Cyclopentylmethyl, Cyclopentylethyl, Cyclopentylpropyl, Cyclopentyl-cyanomethyl, Cyclopentyl-1-cyanoeth-1-yl, Cyclohexyl, Cyclohexylmethyl, Cyclohexylethyl, Cyclohexylpropyl, Cyclohexylcyanomethyl, Cyclohexyl-1-cyanoeth-1-yl, Cycloheptyl, Cycloheptylmethyl, Cycloheptylethyl, Cycloheptylpropyl, Cycloheptyl-cyanomethyl oder Cycloheptyl-1-cyanoeth-1-yl, oder
für Tetrahydrofuran, Tetrahydrothiophen, Tetrahydropyran oder Tetrahydrothiopyran, wobei jeder dieser Reste einfach bis dreifach, gleichartig oder verschieden substituiert sein kann durch Methyl, Ethyl, n- oder i-Propyl, Fluor, Chlor, Brom, Trifluormethyl, Difluormethyl, Chlordifluormethyl, Bromdifluormethyl, Chlormethyl, Dichlormethyl, Cyano, Methylendioxo, Ethylendioxo, Methylcyclopentyl, Methylcyclohexyl, Ethylcyclopentyl, Ethylcyclohexyl, Phenyl, Chlorphenyl, Dichlorphenyl, Fluorphenyl, Difluorphenyl, Chlorfluorphenyl, Methylphenyl, Dimethylphenyl, Chlormethylphenyl, Fluormethylphenyl, Trifluormethylphenyl, Chlor-trifluormethylphenyl oder Fluor-trifluormethyl, oder
für Phenyl, Naphthyl, Phenylalkyl oder Naphthylalkyl mit 1 bis 4 Kohlenstoffatomen im geradkettigen oder verzweigten, gegebenenfalls durch Cyano substituierten Alkylteil, wobei jeder dieser Reste im Arylteil einfach bis dreifach, gleichartig oder verschieden substituiert sein kann durch Fluor, Chlor, Brom, Hydroxy, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Cyclopropyl, Cyclopentyl, Cyclohexyl, Cycloheptyl, Trifluormethyl, Trifluormethoxy, Trifluormethylthio oder gebenenfalls ein- bis dreifach, gleich oder verschieden durch Fluor, Chlor, Brom, Methyl und/oder Ethyl substituiertes Phenyl, oder
für 5- oder 6-gliedriges Hetaryl, oder
für 5- oder 6-gliedriges Hetarylalkyl mit 1 bis 4 Kohlenstoffatomen im geradkettigen oder verzweigten, gegebenenfalls durch Cyano substituierten Alkylteil, wobei der Hetarylteil jeweils 1 bis 3 Heteroatome, wie Sauerstoff, Schwefel und/oder Stickstoff, enthält und einfach bis dreifach, gleichartig oder verschieden substituiert sein kann durch Methyl, Ethyl, n- oder i-Propyl, n-, i-, s- oder t-Butyl, Fluor, Chlor, Brom, Cyano, Trifluormethyl, Difluormethyl, Chlordifluormethyl, Bromdifluormethyl, Chlormethyl oder Dichlormethyl.

$R^7$ steht besonders bevorzugt für Methyl, Ethyl, n- oder i-Propyl, n-, i-, s- oder t-Butyl, n- oder i-Pentyl und n- oder i-Hexyl, wobei jeder dieser Reste einfach bis dreifach, gleichartig oder verschieden substituiert sein kann durch Cyano, Amino, Hydroxy, Carboxy, Methoxy, Ethoxy, n- oder i-Propoxy, n-, i-, s- oder t-Butoxy, Acetyl, Ethylcarbonyl, n- oder i-Propylcarbonyl und n-, i-, s- oder t-Butylcarbonyl, oder
für jeweils gegebenenfalls ein- bis dreifach, gleich oder verschieden durch Fluor und/oder Chlor substituiertes Allyl, n- oder i-Butenyl, n- oder i-Pentenyl und n- oder i-Hexenyl, oder
für Propargyl, n- oder i-Butinyl, n- oder i-Pentinyl und n- oder i-Hexinyl, oder
für Benzyl, das im Phenylteil einfach bis dreifach, gleichartig oder verschieden substituiert sein kann durch Fluor, Chlor, Brom, Methyl, Ethyl, n- oder i-Propyl und n-, i-, s- oder t-Butyl.

$R^8$ steht besonders bevorzugt für Wasserstoff, oder
für Methyl, Ethyl, n- oder i-Propyl, n-, i-, s- oder t-Butyl, n- oder i-Pentyl und n- oder i-Hexyl, wobei jeder dieser Reste einfach bis dreifach, gleichartig oder verschieden substituiert sein kann durch Cyano, Amino, Hydroxy, Carboxy, Methoxy, Ethoxy, n- oder i-Propoxy, n-, i-, s- oder t-Butoxy, Acetyl, Ethylcarbonyl, n- oder i-Propylcarbonyl und n-, i-, s- oder t-Butylcarbonyl, oder
für jeweils gegebenenfalls ein- bis dreifach, gleich oder verschieden durch Fluor und/oder Chlor substituiertes Allyl, n- oder i-Butenyl, n- oder i-Pentenyl und n- oder i-Hexenyl, oder
für Propargyl, n- oder i-Butinyl, n- oder i-Pentinyl und n- oder i-Hexinyl, oder
für Benzyl, das im Phenylteil einfach bis dreifach, gleichartig oder verschieden substituiert sein kann durch Fluor, Chlor, Brom, Methyl, Ethyl, n- oder i-Propyl und n-, i-, s- oder t-Butyl.

$R^7$ und $R^8$ stehen außerdem besonders bevorzugt für zweifach verknüpftes Alkandiyl mit 4 oder 5 $CH_2$-Gruppen, wobei gegebenenfalls eine $CH_2$-Gruppe durch O oder S ersetzt ist.

Die erfindungsgemäß verwendbaren Dibrom-thiophen-carbonsäure-Derivate der Formel (I) sind teilweise bekannt (vgl. z.B. Chem. Ber. 18(1885), 2312; DE-OS 39 01 074; US 3 536 473 EP 0 258 790 JP-OS 62-138, 489 und J. Chem. Soc. Perkin Trans. 1 (1973) 1766, 1772, 1773).

Neu sind die Dibrom-thiophen-carbonsäure-Derivate der Formel

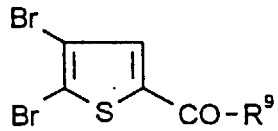

(I-A),

in welcher

$R^9$ für die Gruppierung $-OR^{10}$ steht,
worin

$R^{10}$ für geradkettiges oder verzweigtes Alkyl mit 1 bis 12 Kohlenstoffatomen, steht das einfach bis fünffach, gleichartig oder verschieden substituiert ist durch Halogen, Cyano, Amino, Hydroxy, Mercapto, Carboxy, Nitro, jeweils geradkettiges oder verzweigtes Alkoxy, Alkylthio oder Alkoxycarbonyl mit jeweils 1 bis 6 Kohlenstoffatomen in den einzelnen Alkylteilen, oder
für gegebenenfalls einfach bis fünffach, gleich oder verschieden durch Halogen substituiertes geradkettiges oder verzweigtes Alkenyl mit 3 bis 6 Kohlenstoffatomen steht,
oder
für Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im geradkettigen oder verzweigten, gegebenenfalls durch Cyano substituierten Alkylteil steht wobei der Cycloalkylteil der beiden genannten Reste einfach bis fünffach, gleichartig oder verschieden substituiert sein kann durch geradkettiges oder verzweigtes Alkyl mit 1 bis 4 Kohlenstoffatomen, Halogen, geradkettiges oder verzweigtes Halogenalkyl mit 1 bis 4 Kohlenstoffatomen und 1 bis 5 gleichen oder verschiedenen Halogenatomen, Cyano, Alkylendioxo mit 1 bis 3 Kohlenstoffatomen, gegebenenfalls einfach bis dreifach, gleichartig oder verschieden durch Alkyl mit 1 bis 4 Kohlenstoffatomen substituiertes Cycloalkyl mit 3 bis 7 Kohlenstoffatomen und/oder durch Phenyl, welches seinerseits einfach bis dreifach, gleichartig oder verschieden substituiert sein kann durch geradkettiges oder verzweigtes Alkyl mit 1 bis 4 Kohlenstoffatomen, Halogen und geradkettiges oder verzweigtes Halogenalkyl mit 1 bis 4 Kohlenstoffatomen und 1 bis 5 gleichen oder verschiedenen Halogenatomen, oder
für Aryl mit 6 bis 10 Kohlenstoffatomen oder Aralkyl mit 6 bis 10 Kohlenstoffatomen im Arylteil und 1 bis 4 Kohlenstoffatomen im geradkettigen oder verzweigten, gegebenenfalls durch Cyano substituierten Alkylteil steht wobei jeder der beiden Reste im Arylteil einfach bis dreifach, gleichartig oder verschieden substituiert sein kann durch Halogen, Hydroxy, Cyano, Nitro, jeweils geradkettiges oder verzweigtes Alkyl, Alkoxy oder Alkylthio mit jeweils 1 bis 4 Kohlenstoffatomen, Cycloalkyl mit 3 bis 7 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 5 gleichen oder verschiedenen Halogenatomen und/oder durch Phenyl, das seinerseits einfach bis dreifach, gleichartig oder verschieden substituiert sein kann durch Halogen und/oder Alkyl mit 1 bis 4 Kohlenstoffatomen, Dibrom-thiophen-carbonsäure-Derivate der Formel (I-A) lassen sich herstellen, indem man Dibrom-thiophen-carbonsäure-halogenide der Formel

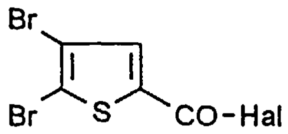

(II),

in welcher

Hal für Halogen steht,
mit Alkoholen der Formel.

$$HO\text{-}R^{10} \quad (IV)$$

in welcher

$R^{10}$ die oben angegebenen Bedeutungen hat, gegebenenfalls in Gegenwart eines Verdünnungsmittels und gegebenenfalls in Gegenwart eines Reaktionshilfsmittels sowie gegebenenfalls in Gegenwart eines Katalysators umsetzt.

In analoger Weise lassen sich die bereits bekannten Dibrom-thiophen-carbonsäure-Derivate der Formel (I) herstellen.

Die neuen Dibrom-thiophen-carbonsäure-Derivate sind durch die Formel (I-A) allgemein definiert.

$R^{10}$ steht bevorzugt für geradkettiges oder verzweigtes Alkyl mit 1 bis 8 Kohlenstoffatomen, das einfach bis vierfach, gleichartig oder verschieden substituiert ist durch Fluor, Chlor, Cyano, Amino, Hydroxy, Mercapto, jeweils geradkettiges oder verzweigtes Alkoxy und Alkylthio mit jeweils 1 bis 6 Kohlenstoffatomen, oder
für gegebenenfalls einfach bis dreifach, gleich oder verschieden durch Fluor, Chlor und/oder Brom substituiertes geradkettiges oder verzweigtes Alkenyl mit 3 bis 6 Kohlenstoffatomen, oder
für Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im geradkettigen oder verzweigten, gegebenenfalls durch Cyano substituierten Alkylteil, wobei der Cycloalkylteil der beiden genannten Reste einfach bis vierfach, gleichartig oder verschieden substituiert sein kann durch geradkettiges oder verzweigtes Alkyl mit 1 bis 4 Kohlenstoffatomen, Halogen, geradkettiges oder verzweigtes Halogenalkyl mit 1 bis 4 Kohlenstoffatomen und 1 bis 3 gleichen oder verschiedenen Halogenatomen, Cyano, Alkylendioxo mit 1 oder 2 Kohlenstoffatomen, gegebenenfalls durch Methyl oder Ethyl substituiertes Cyclohexyl oder Cyclopentyl und/oder durch Phenyl, das seinerseits einfach oder zweifach, gleichartig oder verschieden substituiert sein kann durch Methyl, Ethyl, Fluor, Chlor und/oder Halogenalkyl mit 1 oder 2 Kohlenstoffatomen und 1 bis 3 gleichen oder verschiedenen Halogenatomen, oder
für Phenyl, Naphthyl, Phenylalkyl oder Naphthylalkyl mit 1 bis 4 Kohlenstoffatomen im geradkettigen oder verzweigten, gegebenenfalls durch Cyano substituierten Alkylteil, wobei jeder der vier genannten Reste im Arylteil einfach bis dreifach, gleichartig oder verschieden substituiert sein kann durch Halogen, Hydroxy, Cyano, Nitro, jeweils geradkettiges oder verzweigtes Alkyl, Alkoxy oder Alkylthio mit jeweils 1 bis 4 Kohlenstoffatomen, Cycloalkyl mit 3 bis 7 Kohlenstoffatomen, jeweils geradkettiges oder verzweigtes Halogenalkyl, Halogenalkoxy oder Halogenalkylthio mit jeweils 1 bis 2 Kohlenstoffatomen und 1 bis 5 gleichen oder verschiedenen Halogenatomen und/oder gegebenenfalls einfach bis dreifach, gleich oder verschieden durch Halogen und/oder Alkyl mit 1 bis 2 Kohlenstoffatomen substituiertes Phenyl.

$R^{10}$ steht besonders bevorzugt für Methyl, Ethyl, n- oder i-Propyl, n-, i-, s- oder t-Butyl, n- oder i-Pentyl, n- oder i-Hexyl, n- oder i-Heptyl und n- oder i-Octyl, wobei jeder dieser Reste einfach bis dreifach, gleichartig oder verschieden substituiert ist durch Fluor, Chlor, Cyano, Amino, Hydroxy, Mercapto, Methoxy, Ethoxy, n- oder i-Propoxy, n-, i-, s- oder t-Butoxy, Methylthio, Ethylthio, n- oder i-Propylthio, oder
für jeweils gegebenenfalls einfach bis dreifach, gleich oder verschieden durch Fluor und/oder Chlor substituiertes Allyl, n- oder i-Butenyl, n- oder i-Pentenyl, n- oder i-Hexenyl, oder
für jeweils gegebenenfalls im Cycloalkylteil ein- bis vierfach, gleich oder verschieden durch Methyl, Ethyl, n- oder i-Propyl, Fluor, Chlor, Brom, Trifluormethyl, Difluormethyl, Chlordifluormethyl, Bromdifluormethyl, Chlormethyl, Dichlormethyl, Cyano, Methylendioxo, Ethylendioxo, Methylcyclopentyl, Methylcyclohexyl, Ethylcyclopentyl, Ethylcyclohexyl, Phenyl, Chlorphenyl, Dichlorphenyl, Fluorphenyl, Difluorphenyl, Chlorfluorphenyl,

Methylphenyl, Dimethylphenyl, Chlormethylphenyl, Fluormethylphenyl, Trifluormethylphenyl, Chlor-trifluormethylphenyl oder Fluor-trifluormethyl substituiertes Cyclopropyl, Cyclopropylmethyl, Cyclopropylethyl, Cyclopropylpropyl, Cyclopropyl-1-cyanoeth-1-yl, Cyclopropyl-cyanomethyl, Cyclopentyl, Cyclopentylmethyl, Cyclopentylethyl, Cyclopentylpropyl, Cyclopentyl-cyanomethyl, Cyclopentyl-1-cyanoeth-1-yl, Cyclohexyl, Cyclohexylmethyl, Cyclohexylethyl, Cyclohexylpropyl, Cyclohexyl-cyanomethyl, Cyclohexyl-1-cyanoeth-1-yl, Cycloheptyl, Cycloheptylmethyl, Cycloheptylethyl, Cycloheptylpropyl, Cycloheptylcyanomethyl oder Cycloheptyl-1-cyanoeth-1-yl, oder

für Phenyl, Naphthyl, Phenylalkyl oder Naphthylalkyl mit 1 bis 4 Kohlenstoffatomen im geradkettigen oder verzweigten, gegebenenfalls durch Cyano substituierten Alkylteil, wobei jeder dieser Reste im Arylteil einfach bis dreifach, gleichartig oder verschieden substituiert sein kann durch Fluor, Chlor, Brom, Hydroxy, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Cyclopropyl, Cyclopentyl, Cyclohexyl, Cycloheptyl, Trifluormethyl, Trifluormethoxy, Trifluormethylthio oder gegebenenfalls ein- bis dreifach, gleich oder verschieden durch Fluor, Chlor, Brom, Methyl und/oder Ethyl substituiertes Phenyl.

Verwendet man beispielsweise 4,5-Dibrom-thiophen-2-carbonsäurebromid und Cyclohexanol als Ausgangsstoffe, so läßt sich der Reaktionsablauf des erfindungsgemäßen Verfahrens durch das folgende Formelschema darstellen:

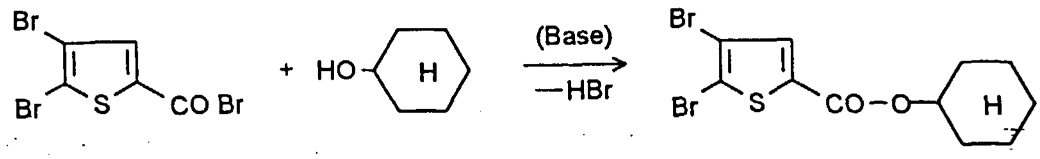


Die zur Durchführung des erfindungsgemäßen Verfahrens als Ausgangsstoffe benötigten Dibrom-thiophencarbonsäurehalogenide sind durch die Formel (II) allgemein definiert. In dieser Formel (II) steht Hal vorzugsweise für Chlor oder Brom.

Die Dibrom-thiophen-carbonsäurehalogenide der Formel (II) sind bekannt (vgl. z. B. EP-OS 0 450 355; US-PS 3.303.201; DE-OS 12 01 952; GB-PS 1085 974).

Die zur Durchführung des erfindungsgemäßen Verfahrens weiterhin als Ausgangsstoffe benötigten Alkohole sind durch die Formel (IV) allgemein definiert. In dieser Formel (IV) steht $R^{10}$ vorzugsweise für diejenigen Reste, die bereits im Zusammenhang mit der Beschreibung der erfindungsgemäß verwendbaren Verbindungen der Formel (I-A) als bevorzugt für diesen Substituenten genannt wurden.

Die Alkohole der Formel (IV) sind allgemein bekannte Verbindungen der organischen Chemie oder erhältlich in Analogie zu allgemein bekannten Verfahren.

Als Verdünnungsmittel zur Durchführung des erfindungsgemäßen Verfahrens kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat, Natriumhydrogencarbonat, ferner Ammoniumhydroxid, Ammoniumacetat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren wird außerdem gegebenenfalls in Gegenwart eines geeigneten Katalysators durchgeführt. Als solche kommen insbesondere Kupfer-(I)-Salze, wie beispielsweise Kupfer-(I)-chlorid infrage. Hierbei kann der Zusatz von katalytischen Mengen eines geeigneten Phasentransferkatalysators, wie beispielsweise 15-Krone-5, 18-Krone-6 oder Tris-[2-(2-methoxyethoxy)-ethyl]-amin von Vorteil sein.

Die Reaktionstemperaturen können bei der Durchführung des erfindungsgemäßen Verfahrens in einem größeren Bereich variiert werden. Im allgemeinen arbeitet man bei Temperaturen zwischen -20°C und +180°C, vorzugsweise bei Temperaturen zwischen 0°C und +150°C.

Das erfindungsgemäße Verfahren wird üblicherweise unter Normaldruck durchgeführt. Es ist jedoch auch möglich, unter erhöhtem oder vermindertem Druck zu arbeiten.

Zur Durchführung des erfindungsgemäßen Verfahrens setzt man pro Mol an Dibrom-thiophen-carbonsäurehalogenid der Formel (II) im allgemeinen 1,0 bis 3,0 Mol, vorzugsweise 1,0 bis 1,5 Mol an Alkohol der Formel (IV) und gegebenenfalls 0,1 bis 3,0 Mol, vorzugsweise 0,5 bis 1,5 Mol an als Reaktionshilfsmittel verwendeter Base ein. Die Reaktionsdurchführung, Aufarbeitung und Isolierung der Reaktionsprodukte erfolgt jeweils nach allgemein bekannten Verfahren (vgl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie ($^1$H-NMR).

Die erfindungsgemäßen Wirkstoffe der Formel (I) weisen eine starke Wirkung gegen Mikroorganismen auf und können zur Bekämpfung von unerwünschten Mikroorganismen praktisch eingesetzt werden. Die Wirkstoffe sind insbesondere für den Gebrauch als Fungizide und Resistenzinduktoren geeignet.

Fungizide werden im Pflanzenschutz eingesetzt zur Bekämpfung von *Plasmodiophoromycetes, Oomycetes, Chytridiomycetes, Zygomycetes, Ascomycetes, Basidiomycetes und Deuteromycetes.*

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechlera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellucularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Krankheiten im Obst- und Gemüsebau, wie beispielsweise gegen den Erreger der Tomatenbraunfäule (Phytophthora infestans) oder gegen den Erreger des Apfelschorfs (Venturia inaequalis) oder gegen den Erreger des echten Mehltaus an Reben (Uncinula necator) oder gegen den Erreger des Rostes an der Bohne (Uromyces appendiculatus) oder zur Bekämpfung von Reis-Krankheiten, wie beispielsweise gegen den Erreger der Reisfleckenkrankheit (Pyricularia oryzae) oder zur Bekämpfung von echten Mehltaupilzen an Getreide, Äpfeln und Gurken sowie zur Bekämpfung von *Alternaria solani* und *Peronospora brassicae* eingesetzt werden.

Die erfindungsgemäßen Wirkstoffe weisen des weiteren eine starke resistenz-induzierende Wirkung in

Pflanzen auf. Sie eignen sich daher zur Erzeugung von Resistenz in Pflanzen gegen Befall durch unerwünschte Mikroorganismen.

Unter resistenz-induzierenden Stoffen sind im vorliegenden Zusammenhang solche Substanzen zu verstehen, die einerseits bei direkter Einwirkung auf die unerwünschten Mikroorganismen nur eine geringe Aktivität zeigen, andererseits aber in der Lage sind, das Abwehrsystem von Pflanzen so zu stimulieren, daß die behandelten Pflanzen bei nachfolgender Inokulation mit unerwünschten Mikroorganismen weitergehende Resistenz gegen diese Mikroorganismen entfalten.

Unter unerwünschten Mikroorganismen sind im vorliegenden Fall phytophatogene Pilze, Bakterien und Viren zu verstehen. Die erfindungsgemäßen Stoffe können also eingesetzt werden, um in Pflanzen innerhalb eines gewissen Zeitraumes nach der Behandlung Resistenz gegen den Befall durch die genannten Schaderreger zu erzeugen. Der Zeitraum, innerhalb dessen Resistenz herbeigeführt wird, erstreckt sich im allgemeinen von 1 bis 10 Tage, vorzugsweise 1 bis 7 Tage nach der Behandlung der Pflanzen mit den Wirkstoffen.

Zu den unerwünschten Mikroorganismen im Pflanzenschutz gehören Pilze aus den Klassen der *Plasmodiophoromycetes, Oomycetes, Chytridiomycetes, Zygomycetes, Ascomycetes, Basidiomycetes und Deuteromycetes.*

Die Wirkstoffe können in Abhängigkeit von ihren jeweiligen physikalischen und/oder chemischen. Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächen-aktiven Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlen-wasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokusnußschalen, Maiskolben und Tabakstengel; als Emulgier- und/oder schaumerzeugende Mittel kommen in Frage: z.B. nichtionogene ond anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexföromige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetable Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennärstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Besonders günstige Mischpartner sind z.B. die folgenden Verbindungen:

**Fungizide:**

2-Aminobutan;2-Anilino-4-methyl-6-cyclopropyl-pyrimidin;2',6'-Dibromo-2-methyl-4'-trifluoromethoxy-4'-trifluoro-methyl-1,3-thiazol-5-carboxanilid;2,6-Dichloro-N-(4-trifluoromethylbenzyl)-benzamid; (E)-2-Methoxyimino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-o-tolyl]-acetat; 2-Phenylphenol (OPP), Aldimorph, Ampropylfos, Anilazin, Azaconazol,

Benalaxyl, Benodanil, Benomyl, Binapacryl, Biphenyl, Bitertanol, Blasticidin-S, Bromuconazole, Bupirimate, Buthiobate,

Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,

Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,

Edifenphos, Epoxyconazole, Ethirimol, Etridiazol,

Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,

Guazatine,

Hexachlorbenzol, Hexaconazol, Hymexazol,

Imazalil, Imibenconazol, Iminoctadin, Iprobenfos (IBP), Iprodion, Isoprothiolan,

Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,

Mancopper, Mancozeb, Maneb, Mepanipyrim, Mepronil, Metalaxyl, Metconazol, Methasulfocarb, Methfuroxam, Metiram, Metsulfovax, Myclobutanil,

Nickel dimethyldithiocarbamat, Nitrothal-isopropyl, Nuarimol,

Ofurace, Oxadixyl, Oxamocarb, Oxycarboxin,

Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,

Quintozen (PCNB),

Schwefel und Schwefel-Zubereitungen,

Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,

Validamycin A, Vinclozolin,

Zineb, Ziram

**Bakterizide:**

Bromopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,

Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,

Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,

Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,

Edifenphos, Emamectin, Esfenvalerat, Ethiofencarb, Ethion, Ethofenprox, Ethoprophos, Etrimphos,

Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,

HCH, Heptenophos, Hexaflumuron, Hexythiazox,
Imidacloprid, Iprobenfos, Isazophos, Isofenphos, Isoprocarb, Isoxathion, Ivemectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden.

Die Anwendung geschieht in üblicher Weise, z.B. durch Gießen, Verspritzen, Versprühen, Verstreuen, Verstäuben, Verschäumen, Bestreichen usw.

Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut von Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung weden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Bei der Anwendung als Resistenzinduktoren können die Wirkstoffkonzentrationen für die Behandlung von Pflanzenteilen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Die Herstellung erfindungsgemäßer Verbindungen wird durch das folgende Beispiel veranschaulicht.

**Herstellungsbeispiel**

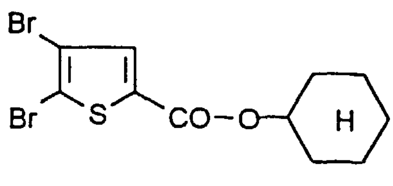

5 g (0,014 Mol) 4,5-Dibrom-thiophen-2-carbonsäurebromid in 40 ml Chloroform werden mit 1,5 g (0,015 Mol) Cyclohexanol versetzt. Diese Reaktionsmischung wird tropfenweise mit 2,1 g (0,021 Mol) Triethylamin in 5 ml Chloroform versetzt und anschließend 2 Stunden bei Raumtemperatur nachgerührt.
Anschließend wird mit Wasser versetzt, die organische Phase abgetrennt, mit Wasser gewaschen und unter vermindertem Druck eingeengt. Der Rückstand wird säulenchromatographisch (Kieselgel / Methylenchlorid) gereinigt. Man

erhält 4,23 g (81,1 % der Theorie) 4,5-Dibrom-thiophen-2-carbonsäure-cyclohexylester vom Berechnungsindex $n_D^{20}$ = 1,5845.

In Beispielhaft seien in der folgenden Tabelle die Säure und Ester aufgeführt, die erfindungsgemäß verwendbar sind. Sie können gegebenenfalls gemäß den oligen Angaben zur Herstellung erhalten werden.

**Tabelle 1:**

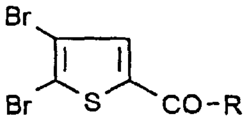

(I)

| Beispiel-Nr. | R | Physikalische Konstante |
|---|---|---|
| 2 | $-O-CH_2CH_2OCH_3$ | Fp. 37°C |
| 3 | -OH | Fp. 222-224°C |
| 4 | $-OCH_3$ | Fp. 73-75°C |
| 5 | $OC_3H_7$-n | $n_D^{20}$ = 1,5759 |
| 6 | $OC_3H_7$-i | $n_D^{20}$ = 1,5764 |
| 7 | 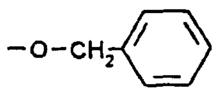 | Fp. 70-72°C |
| 8 | 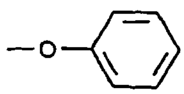 | Fp. 113-115°C |
| 9 | $-O-CH_2-CH=CH_2$ | $n_D^{20}$ = 1,5962 |
| 10 | $-O-CH_2-C{\equiv}CH$ | Fp. 54°C |
| 11 | 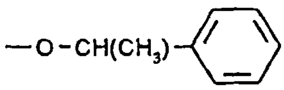 | $n_D^{20}$ = 1,5981 |
| 12 | 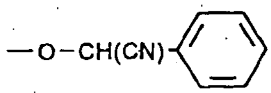 | Fp. 65°C |
| 13 | $-OC_4H_9$-n | $n_D^{20}$ = 1,5599 |
| 14 | $-O-CH(CH_3)-C_4H_9$-t | $^1H$-NMR ($CDCl_3$) δ = 0,96ppm (3H) |
| 15 | $-O-CH_2-C_3H_7$-i | $n_D^{20}$ = 1,5599 |
| 16 | $-O-CH(CH_3)-C{\equiv}CH$ | Fp. 38°C |
| 17 | $-O-CH(CH_3)-CH=CH_2$ | $n_D^{20}$ = 1.5819 |
| 18 | $-O-CH_2CH_2-C(CH_3)=CH_2$ | $n_D^{20}$ = 1,5749 |

**Tabelle 1:** (Fortsetzung)

| Beispiel-Nr. | R | Physikalische Konstante |
|---|---|---|
| 19 | $-O-CH(CH_3)C_2H_5$ | $n_D^{20} = 1.5635$ |

## Anwendungsbeispiele

### Beispiel A

**Pyricularia-Test (Reis) / protektiv**

| | | |
|---|---|---|
| Lösungsmittel | 12,5 | Gewichtsteile Aceton |
| Emulgator | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Antrocknen des Spritzbelages werden die Pflanzen mit einer wäßrigen Sporensuspension von Pyricularia oryzae inokuliert. Anschließend werden die Pflanzen in einem Gewächshaus bei 100 % relativer Luftfeuchtigkeit und 25°C aufgestellt.

4 Tage nach der Inokulation erfolgt die Auswertung des Krankheitsbefalls.

Wirkstoffe, Wirkstoffkonzentrationen und Versuchsergebnisse für die erfindungsgemäße Verwendung gehen aus der folgenden Tabelle hervor.

## Tabelle A

**Pyricularia-Test (Reis) / protektiv**

| Wirkstoff | Wirkstoffkonzentration in % | Wirkungsgrad in % der unbehandelten Kontrolle |
|---|---|---|
| Erfindungsgemäß | | |
| 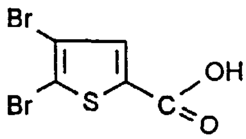 (3) | 0,025 | 70 |
| 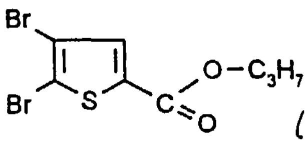 (5) | 0,025 | 70 |
| 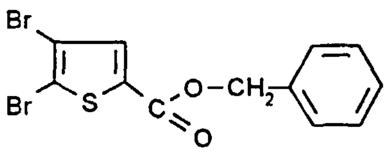 (7) | 0,025 | 80 |

**Beispiel B**

**Uncinula-Test (Rebe) / protektiv**

| | | |
|---|---|---|
| Lösungsmittel | 4,7 | Gewichtsteile Aceton |
| Emulgator | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes *Unicinula necator* bestäubt.

Die Pflanzen werden anschließend bei 23 bis 24°C und bei einer relativen Luftfeuchtigkeit von ca. 75 % im Gewächshaus aufgestellt.

14 Tage nach der Inokulation erfolgt die Auswertung.

Wirkstoffe, Wirkstoffkonzentrationen und Versuchsergebnisse für die erfindungsgemäße Verwendung gehen aus der folgenden Tabelle hervor.

**Tabelle B**

**Uncinula-Test (Rebe) / protektiv**

| Wirkstoff | Wirkungsgrad in % der unbehandelten Kontrolle bei einer Wirkstoffkonzentration von 25 ppm. |
|---|---|
| 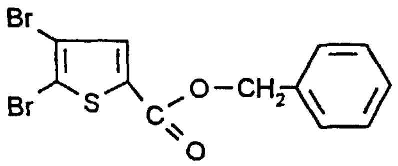 (7) | 100 |

**Patentansprüche**

1. Verwendung von Dibrom-thiophen-carbonsäure-Derivaten der Formel

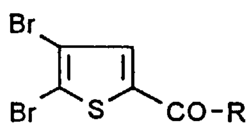 (I),

in welcher

R für die Gruppierung $-XR^1$ steht, worin

X für Sauerstoff oder Schwefel steht und

$R^1$ für Wasserstoff,
oder geradkettiges oder verzweigtes Alkyl mit 1 bis 12 Kohlenstoffatomen steht, das einfach bis fünffach, gleichartig oder verschieden substituiert sein kann durch Halogen, Cyano, Amino, Hydroxy, Mercapto, Carboxy, Nitro, jeweils geradkettiges oder verzweigtes Alkoxy, Alkylthio oder Alkoxycarbonyl mit jeweils 1 bis 6 Kohlenstoffatomen in den einzelnen Alkylteilen, sowie die Gruppierung -$NR^7R^8$, oder

für jeweils gegebenenfalls einfach bis fünffach, gleich oder verschieden durch Halogen substituiertes geradkettiges oder verzweigtes Alkenyl und Alkinyl mit 3 bis 6 Kohlenstoffatomen steht, oder für Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im geradkettigen oder verzweigten, gegebenenfalls durch Cyano substituierten Alkylteil steht, wobei der Cycloalkylteil der beiden genannten Reste einfach bis fünffach, gleichartig oder verschieden substituiert sein kann durch geradkettiges oder verzweigtes Alkyl mit 1 bis 4 Kohlenstoffatomen, Halogen, geradkettiges oder verzweigtes Halogenalkyl mit 1 bis 4 Kohlenstoffatomen und 1 bis 5 gleichen oder verschiedenen Halogenatomen, Cyano, Alkylendioxo mit 1 bis 3 Kohlenstoffatomen, gegebenenfalls einfach bis dreifach, gleichartig oder verschieden durch Alkyl mit 1 bist 4 Kohlenstoffatomen substituiertes Cycloalkyl mit 3 bis 7 Kohlenstoffatomen und/oder durch Phenyl, welches seinerseits einfach bis dreifach, gleichartig oder verschieden substituiert sein kann durch geradkettiges oder verzweigtes Alkyl mit 1 bis 4 Kohlenstoffatomen, Halogen und geradkettiges oder verzweigtes Halogenalkyl mit 1 bis 4 Kohlenstoffatomen und 1 bis 5 gleichen oder verschiedenen Halogenatomen, oder

für Tetrahydrofuran, Tetrahydrothiophen, Tetrahydropyran oder für Tetrahydrothiopyran steht, wobei jeder dieser Reste einfach bis dreifach, gleichartig oder verschieden substituiert sein kann durch geradkettiges oder verzweigtes Alkyl mit 1 bis 4 Kohlenstoffatomen, Halogen, geradkettiges oder verzweigtes Halogenalkyl mit 1 bis 4 Kohlenstoffatomen und 1 bis 5 gleichen oder verschiedenen Halogenatomen, Cyano, Alkylendioxo mit 1 bis 3 Kohlenstoffatomen, gegebenenfalls einfach bis dreifach, gleichartig oder verschieden durch Alkyl mit 1 bis 4 Kohlenstoffatomen substituiertes Cycloalkyl mit 3 bis 7 Kohlenstoffatomen und/oder durch Phenyl, welches seinerseits einfach bis dreifach, gleichartig oder verschieden substituiert sein kann durch geradkettiges oder verzweigtes Alkyl mit 1 bis 4 Kohlenstoffatomen, Halogen und geradkettiges oder verzweigtes Halogenalkyl mit 1 bis 4 Kohlenstoffatomen und 1 bis 5 gleichen oder verschiedenen Halogenatomen, oder

für Aryl mit 6 bis 10 Kohlenstoffatomen oder Aralkyl mit 6 bis 10 Kohlenstoffatomen im Arylteil und 1 bis 4 Kohlenstoffatomen im geradkettigen oder verzweigten, gegebenenfalls durch Cyano substituierten Alkylteil steht, wobei jeder der beiden Reste im Arylteil einfach bis dreifach, gleichartig oder verschieden substituiert sein kann durch Halogen, Hydroxy, Cyano, Nitro, jeweils geradkettiges oder verzweigtes Alkyl, Alkoxy oder Alkylthio mit jeweils 1 bis 4 Kohlenstoffatomen, Cycloalkyl mit 3 bis 7 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 5 gleichen oder verschiedenen Halogenatomen und/oder durch Phenyl, das seinerseits einfach bis dreifach, gleichartig oder verschieden substituiert sein kann durch Halogen und/oder Alkyl mit 1 bis 4 Kohlenstoffatomen, oder

für 5- oder 6-gliedriges Hetaryl oder für 5- oder 6-gliedriges Hetarylalkyl mit 1 bis 6 Kohlenstoffatomen im geradkettigen oder verzweigten, gegebenenfalls durch Cyano substituierten Alkylteil steht, wobei der Hetarylteil jeweils 1 bis 3 Heteroatome, wie Sauerstoff, Schwefel und/oder Stickstoff, enthält und einfach bis dreifach, gleichartig oder verschieden substituiert sein kann durch geradkettiges oder verzweigtes Alkyl mit 1 bis 6 Kohlenstoffatomen, Halogen, Cyano und/oder geradkettiges oder verzweigtes Halogenalkyl mit 1 bis 4 Kohlenstoffatomen und 1 bis 5 gleichen oder verschiedenen Halogenatomen,

$R^7$ für geradkettiges oder verzweigtes Alkyl mit 1 bis 6 Kohlenstoffatomen steht, das einfach bis fünffach, gleichartig oder verschieden substituiert sein kann durch Halogen, Cyano, Amino, Hydroxy, Mercapto, Nitro, Carboxy, jeweils geradkettiges oder verzweigtes Alkoxy und Alkoxycarbonyl mit jeweils 1 bis 6 Kohlenstoffatomen in den Alkoxyteilen, Alkylamino und Dialkylamino mit 1 bis 6 Kohlenstoffatomen in jedem geradkettigen oder verzweigten Alkylteil, oder

für jeweils gegebenenfalls durch 1 bis 5 gleiche oder verschiedene Halogenatome substituiertes geradkettiges oder verzweigtes Alkenyl oder Alkinyl mit jeweils 3 bis 6 Kohlenstoffatomen steht, oder für Benzyl steht, das im Phenylteil einfach bis dreifach, gleichartig oder verschieden substituiert sein kann durch Halogen, Nitro, Cyano, geradkettiges oder verzweigtes Alkyl mit 1 bis 6 Kohlenstoffatomen, sowie jeweils geradkettiges oder verzweigtes Halogenalkyl, Halogenalkoxy oder Halogenalkylthio mit jeweils 1 bis 4 Kohlenstoffatomen und 1 bis 5 gleichen oder verschiedenen Halogenatomen und

$R^8$ für Wasserstoff oder für geradkettiges oder verzweigtes Alkyl mit 1 bis 6 Kohlenstoffatomen steht, das einfach bis fünffach, gleichartig oder verschieden substituiert sein kann durch Halogen, Cyano, Amino, Hydroxy, Mercapto, Nitro, Carboxy, jeweils geradkettiges oder verzweigtes Alkoxy und Alkoxycarbonyl mit jeweils 1 bis 6 Kohlenstoffatomen in den Alkoxyteilen, Alkylamino und Dialkylamino mit 1 bis 6 Kohlenstoffatomen in jedem geradkettigen oder verzweigten Alkylteil, oder für jeweils gegebenenfalls durch 1 bis 5 gleiche oder verschiedene Halogenatome substituiertes geradkettiges oder verzweigtes Alkenyl oder Alkinyl mit jeweils 3 bis 6 Kohlenstoffatomen steht, oder für Benzyl steht, das im Phenylteil einfach bis dreifach, gleichartig oder verschieden substituiert sein kann durch Halogen, Nitro, Cyano, geradkettiges oder verzweigtes Alkyl mit 1 bis 6 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 5 gleichen oder verschiedenen Halogenatomen, oder

$R^7$ und $R^8$ außerdem gemeinsam für zweifach verknüpftes Alkandiyl mit 4 bis 6 $CH_2$-Gruppen stehen, wobei gegebenenfalls eine $CH_2$-Gruppe durch O oder S ersetzt ist,

zur Bekämpfung von unerwünschten Mikroorganismen.

2. Verwendung gemäß Anspruch 1, dadurch gekennzeichnet, daß als Dibromthiophen-carbonsäure-Derivat die Verbindung der Formel

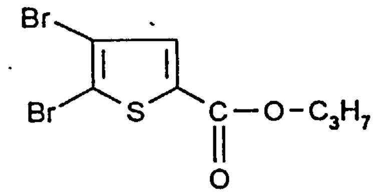

eingesetzt wird.

3. Verwendung gemäß Anspruch 1, dadurch gekennzeichnet, daß als Dibromthiophen-carbonsäure-Derivat die Verbindung der Formel

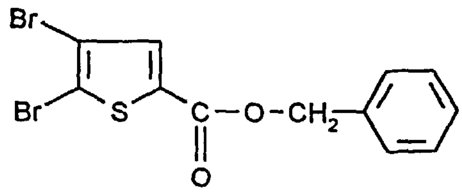

eingesetzt wird.

4. Dibrom-thiophen-carbonsäure-Derivative der Formel

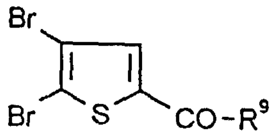

(I-A),

in welcher

$R^9$ für die Gruppierung $-OR^{10}$ steht, worin

$R^{10}$ für geradkettiges oder verzweigtes Alkyl mit 1 bis 12 Kohlenstoffatomen, steht das einfach bis fünffach, gleichartig oder verschieden substituiert ist durch Halogen, Cyano, Amino, Hydroxy, Mercapto, Carboxy, Nitro, jeweils geradkettiges oder verzweigtes Alkoxy, Alkylthio oder Alkoxycarbonyl mit jeweils 1 bis 6 Kohlenstoffatomen in den einzelnen Alkylteilen, oder
für gegebenenfalls einfach bis fünffach, gleich oder verschieden durch Halogen substituiertes geradkettiges oder verzweigtes Alkenyl mit 3 bis 6 Kohlenstoffatomen steht,
oder
für Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im geradkettigen oder verzweigten, gegebenenfalls durch Cyano substituierten Alkylteil steht wobei der Cycloalkylteil der beiden genannten Reste einfach bis fünffach, gleichartig oder verschieden substituiert sein kann durch geradkettiges oder verzweigtes Alkyl mit 1 bis 4 Kohlenstoffatomen, Halogen, geradkettiges oder verzweigtes Halogenalkyl mit 1 bis 4 Kohlenstoffatomen und 1 bis 5 gleichen oder verschiedenen Halogenatomen, Cyano, Alkylendioxo mit 1 bis 3 Kohlenstoffatomen, gegebenenfalls einfach bis dreifach, gleichartig oder verschieden durch Alkyl mit 1 bis 4 Kohlenstoffatomen substituiertes Cycloalkyl mit 3 bis 7 Kohlenstoffatomen und/oder durch Phenyl, welches seinerseits einfach bis dreifach, gleichartig oder verschieden substituiert sein kann durch geradkettiges oder verzweigtes Alkyl mit 1 bis 4 Kohlenstoffatomen, Halogen und geradkettiges oder verzweigtes Halogenalkyl mit 1 bis 4 Kohlenstoffatomen und 1 bis 5 gleichen oder verschiedenen Halogenatomen, oder
für Aryl mit 6 bis 10 Kohlenstoffatomen oder Aralkyl mit 6 bis 10 Kohlenstoffatomen im Arylteil und 1 bis 4 Kohlenstoffatomen im geradkettigen oder verzweigten, gegebenenfalls durch Cyano substituierten Alkylteil steht wobei jeder der beiden Reste im Arylteil einfach bis dreifach, gleichartig oder verschieden substituiert sein kann durch Halogen, Hydroxy, Cyano, Nitro, jeweils geradkettiges oder verzweigtes Alkyl, Alkoxy oder Alkylthio mit jeweils 1 bis 4 Kohlenstoffatomen, Cycloalkyl mit 3 bis 7 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 5 gleichen oder verschiedenen Halogenatomen und/oder durch Phenyl, das seinerseits einfach bis dreifach, gleichartig oder verschieden substituiert sein kann durch Halogen und/oder Alkyl mit 1 bis 4 Kohlenstoffatomen.

**5.** Verfahren zur Herstellung von Dibrom-thiophen-carbonsäure-Derivaten der Formel (I-A) gemäß Anspruch 4, dadurch gekennzeichnet, daß man Dibrom-thiophen-carbonsäure-halogenide der Formel

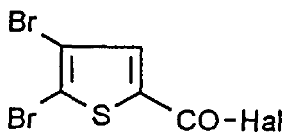

(II),

in welcher

Hal für Halogen steht,
mit Alkoholen der Formel.

$$HO-R^{10} \quad (IV)$$

in welcher

$R^{10}$ die oben angegebenen Bedeutungen hat,
gegebenenfalls in Gegenwart eines Verdünnungsmittels und gegebenenfalls in Gegenwart eines Reaktionshilfsmittels sowie gegebenenfalls in Gegenwart eines Katalysators umsetzt.

**6.** Mikrobizide Mittel, gekennzeichnet durch einen Gehalt an mindestens einem Dibrom-thiophen-carbonsäure-Derivat der Formel (I) gemäß Anspruch 4 neben Streckmitteln und / oder oberflächenaktiven Stoffen.

## Claims

**1.** Use of dibromo-thiophene-carboxylic acid derivatives of the formula

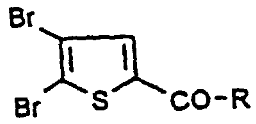

(I),

in which

R represents the group $-XR^1$, in which

X represents oxygen or sulphur, and

$R^1$ represents hydrogen,
or straight-chain or branched alkyl having 1 to 12 carbon atoms, which can be mono- to pentasubstituted, identically or differently, by halogen, cyano, amino, hydroxyl, mercapto, carboxyl, nitro; alkoxy, alkylthio or alkoxycarbonyl, each of which is straight-chain or branched, each having 1 to 6 carbon atoms in the individual alkyl moieties; and also the group $-NR^7R^8$, or
represents straight-chain or branched alkenyl and alkinyl having 3 to 6 carbon atoms, each of which is optionally mono- to pentasubstituted, identically or differently, by halogen, or
cycloalkyl having 3 to 7 carbon atoms or cycloalkylalkyl having 3 to 7 carbon atoms in the cycloalkyl moiety and 1 to 4 carbon atoms in the straight-chain or branched alkyl moiety, which is optionally substituted by cyano, where the cycloalkyl moiety of the two radicals mentioned can be mono- to pentasubstituted, identically or differently, by straight-chain or branched alkyl having 1 to 4 carbon atoms, halogen, straight-chain or branched halogenoalkyl having 1 to 4 carbon atoms and 1 to 5 identical or different halogen atoms, cyano, alkylenedioxo having 1 to 3 carbon atoms, cycloalkyl having 3 to 7 carbon atoms which is optionally mono-to trisubstituted, identically or differently, by alkyl having 1 to 4 carbon atoms, and/or by phenyl, which for its part can be mono- to trisubstituted, identically or differently, by straight-chain or branched alkyl having 1 to 4 carbon atoms, halogen and straight-chain or branched halogenoalkyl having 1 to 4 carbon atoms and 1 to 5 identical or different halogen atoms, or
tetrahydrofuran, tetrahydrothiophene, tetrahydropyran or tetrahydrothiopyran, where each of these radicals can be mono- to trisubstituted, identically or differently, by straight-chain or branched alkyl having 1 to 4 carbon atoms, halogen, straight-chain or branched halogenoalkyl having 1 to 4 carbon atoms and 1 to 5 identical or different halogen atoms, cyano, alkylenedioxo having 1 to 3 carbon atoms, cycloalkyl having 3 to 7 carbon atoms, which is optionally mono- to trisubstituted, identically or differently, by alkyl having 1 to 4 carbon atoms, and/or by phenyl, which for its part can be mono- to trisubstituted, identically or differently, by straight-chain or branched alkyl having 1 to 4 carbon atoms, halogen and straight-chain or branched halogenoalkyl having 1 to 4 carbon atoms and 1 to 5 identical or different halogen atoms, or
aryl having 6 to 10 carbon atoms or aralkyl having 6 to 10 carbon atoms in the aryl moiety and 1 to 4 carbon atoms in the straight-chain or branched alkyl moiety, which is optionally substituted by cyano, where each of the two radicals in the aryl moiety can be mono- to trisubstituted, identically or differently, by halogen, hydroxyl, cyano, nitro; alkyl, alkoxy or alkylthio, each of which is straight-chain or branched, each having 1 to 4 carbon atoms; cycloalkyl having 3 to 7 carbon atoms; halogenoalkyl, halogenoalkoxy or halogenoalkylthio, each of which is straight-chain or branched, each having 1 to 4 carbon atoms and 1 to 5 identical or different halogen atoms; and/or by phenyl which for its part can be mono- to trisubstituted, identically or differently, by halogen and/or alkyl having 1 to 4 carbon atoms, or
5- or 6-membered hetaryl or 5- or 6-membered hetarylalkyl having 1 to 6 carbon atoms in the straight-chain or branched alkyl moiety, which is optionally substituted by cyano, where the hetaryl moiety in each case contains 1 to 3 heteroatoms, such as oxygen, sulphur and/or nitrogen, and can be mono- to trisubstituted, identically or differently, by straight-chain or branched alkyl having 1 to 6 carbon atoms, halogen, cyano and/or straight-chain or branched halogenoalkyl having 1 to 4 carbon atoms

and 1 to 5 identical or different halogen atoms,

$R^7$ represents straight-chain or branched alkyl having 1 to 6 carbon atoms, which can be mono- to pentasubstituted, identically or differently, by halogen, cyano, amino, hydroxyl, mercapto, nitro, carboxyl; alkoxy and alkoxycarbonyl, each of which is straight-chain or branched, each having 1 to 6 carbon atoms in the alkoxy moieties; alkylamino and dialkylamino having 1 to 6 carbon atoms in each straight-chain or branched alkyl moiety, or
straight-chain or branched alkenyl or alkinyl each having 3 to 6 carbon atoms, each of which is optionally substituted by 1 to 5 identical or different halogen atoms, or
benzyl which in the phenyl moiety can be mono- to trisubstituted, identically or differently, by halogen, nitro, cyano, straight-chain or branched alkyl having 1 to 6 carbon atoms, and also halogenoalkyl, halogenoalkoxy or halogenoalkylthio, each of which is straight-chain or branched, each having 1 to 4 carbon atoms and 1 to 5 identical or different halogen atoms and

$R^8$ represents hydrogen, or straight-chain or branched alkyl having 1 to 6 carbon atoms, which can be mono- to pentasubstituted, identically or differently, by halogen, cyano, amino, hydroxyl, mercapto, nitro, carboxyl; alkoxy and alkoxycarbonyl, each of which is straight-chain or branched, each having 1 to 6 carbon atoms in the alkoxy moieties, alkylamino and dialkylamino having 1 to 6 carbon atoms in each straight-chain or branched alkyl moiety, or
straight-chain or branched alkenyl or alkinyl each having 3 to 6 carbon atoms, each of which is optionally substituted by 1 to 5 identical or different halogen atoms, or
benzyl which in the phenyl moiety can be mono- to trisubstituted, identically or differently, by halogen, nitro, cyano, straight-chain or branched alkyl having 1 to 6 carbon atoms, and also halogenoalkyl, halogenoalkoxy or halogenoalkylthio, each of which is straight-chain or branched, each having 1 to 4 carbon atoms and 1 to 5 identical or different halogen atoms, or

$R^7$ and $R^8$ additionally together represent divalent alkanediyl having 4 to 6 $CH_2$ groups, it optionally being possible for a $CH_2$ group to be replaced by O or S,

for controlling undesired microorganisms.

**2.** Use according to Claim 1, characterized in that the dibromo-thiophene-carboxylic acid derivative employed is the compound of the formula

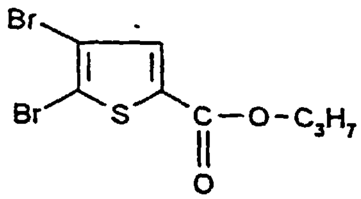

.

**3.** Use according to Claim 1, characterized in that the dibromo-thiophene-carboxylic acid derivative used is the compound of the formula

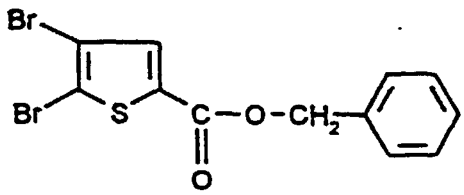

.

**4.** Dibromo-thiophene-carboxylic acid derivatives of the formula

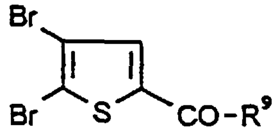

(I-A),

in which

$R^9$ represents the group $-OR^{10}$, in which

$R^{10}$ represents straight-chain or branched alkyl having 1 to 12 carbon atoms, which is mono- to pentasubstituted, identically or differently, by halogen, cyano, amino, hydroxyl, mercapto, carboxyl, nitro, in each case straight-chain or branched alkoxy, alkylthio or alkoxycarbonyl in each case having 1 to 6 carbon atoms in the individual alkyl moieties, or
alkenyl having 3 to 6 carbon atoms, which is optionally mono- to pentasubstituted, identically or differently, by halogen, or
cycloalkyl having 3 to 7 carbon atoms or cycloalkylalkyl having 3 to 7 carbon atoms in the cycloalkyl moiety and 1 to 4 carbon atoms in the straight-chain or branched alkyl moiety, which is optionally substituted by cyano, where the cycloalkyl moiety of the two radicals mentioned can be mono- to pentasubstituted, identically or differently, by straight-chain or branched alkyl having 1 to 4 carbon atoms, halogen, straight-chain or branched halogenoalkyl having 1 to 4 carbon atoms and 1 to 5 identical or different halogen atoms, cyano, alkylenedioxo having 1 to 3 carbon atoms, cycloalkyl having 3 to 7 carbon atoms, optionally mono- to trisubstituted, identically or differently, by alkyl having 1 to 4 carbon atoms, and/or by phenyl, which for its part can be mono- to trisubstituted, identically or differently, by straight-chain or branched alkyl having 1 to 4 carbon atoms, halogen and straight-chain or branched halogenoalkyl having 1 to 4 carbon atoms and 1 to 5 identical or different halogen atoms, or
represents aryl having 6 to 10 carbon atoms or aralkyl having 6 to 10 carbon atoms in the aryl moiety and 1 to 4 carbon atoms in the straight-chain or branched alkyl moiety, which is optionally substituted by cyano, where each of the two radicals in the aryl moiety can be monosubstituted to trisubstituted, identically or differently, by halogen, hydroxyl, cyano, nitro, in each case straight-chain or branched alkyl, alkoxy or alkylthio in each case having 1 to 4 carbon atoms, cycloalkyl having 3 to 7 carbon atoms, in each case straight-chain or branched halogenoalkyl, halogenoalkoxy or halogenoalkylthio in each case having 1 to 4 carbon atoms and 1 to 5 identical or different halogen atoms, and/or by phenyl which for its part can be monosubstituted to trisubstituted, identically or differently, by halogen and/or alkyl having 1 to 4 carbon atoms.

**5.** Process for the preparation of dibromo-thiophene-carboxylic acid derivatives of the formula (I-A) according to Claim 4, characterized in that dibromo-thiophene-carbonyl halides of the formula

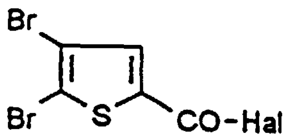

(II),

in which

Hal represents halogen, are reacted with alcohols of the formula

$$HO\text{-}R^{10} \quad (IV)$$

in which

$R^{10}$ has the meanings indicated above, if appropriate in the presence of a diluent and if appropriate in the presence of a reaction auxiliary, and if appropriate in the presence of a catalyst.

**6.** Microbicidal compositions, characterized in that they contain at least one dibromo-thiophene-carboxylic acid derivative of the formula (I) according to Claim 4 in addition to extenders and/or surface-active substances.

**Revendications**

**1.** Utilisation de dérivés d'acide dibromo-thiophène-carboxylique de formule

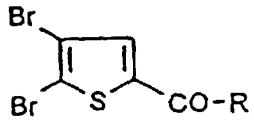

(I),

dans laquelle

R est le groupement $-XR^1$, où
X est l'oxygène ou le soufre et
$R^1$ représente l'hydrogène,
ou un groupe alkyle linéaire ou ramifié ayant 1 à 12 atomes de carbone, qui peut être substitué une à cinq fois identiques ou différentes par un halogène, un radical cyano, amino, hydroxy, mercapto, carboxy, nitro, un radical alkoxy, alkylthio ou alkoxycarbonyle linéaire ou ramifié ayant dans chaque cas 1 à 6 atomes de carbone dans les parties alkyle individuelles, de même que le groupement $-NR^7R^8$, ou bien
un groupe alcényle ou alcynyle linéaire ou ramifié de 3 à 6 atomes de carbone portant chacun le cas échéant 1 à 5 substituants halogéno identiques ou différents, ou bien
un groupe cycloalkyle de 3 à 7 atomes de carbone ou un groupe cycloalkylalkyle ayant 3 à 7 atomes de carbone dans la partie cycloalkyle et 1 à 4 atomes de carbone dans la partie alkyle linéaire ou ramifiée éventuellement substituée par un groupe cyano, la partie cycloalkyle des deux restes mentionnés pouvant être substituée une à cinq fois identiques ou différentes par un radical alkyle linéaire ou ramifié ayant 1 à 4 atomes de carbone, halogéno, halogénalkyle linéaire ou ramifié ayant 1 à 4 atomes de carbone et 1 à 5 atomes d'halogènes identiques ou différents, cyano, alkylènedioxo ayant 1 à 3 atomes de carbone, cycloalkyle de 3 à 7 atomes de carbone portant le cas échéant 1 à 3 substituants alkyle de 1 à 4 atomes de carbone, identiques ou différents
et/ou par un groupe phényle qui peut de son côté être substitué une à trois fois identiques ou différentes par un radical alkyle linéaire ou ramifié ayant 1 à 4 atomes de carbone, halogéno et halogénalkyle linéaire ou ramifié ayant 1 à 4 atomes de carbone et 1 à 5 atomes d'halogènes identiques ou différents, ou bien
un reste tétrahydrofuranne, tétrahydrothiophène, tétrahydropyranne ou un reste tétrahydrothiopyranne, chacun de ces restes pouvant être substitué une à trois fois identiques ou différentes par un radical alkyle linéaire ou ramifié ayant 1 à 4 atomes de carbone, halogéno, halogénalkyle linéaire ou ramifié ayant 1 à 4 atomes de carbone et 1 à 5 atomes d'halogènes identiques ou différents, cyano, alkylènedioxo ayant 1 à 3 atomes de carbone, cycloalkyle de 3 à 7 atomes de carbone éventuellement substitué une à trois fois identiques ou différentes par un radical alkyle ayant 1 à 4 atomes de carbone, et/ou par un groupe phényle qui peut être substitué de son côté une à trois fois identiques ou différentes par un radical alkyle linéaire ou ramifié ayant 1 à 4 atomes de carbone, halogéno et halogénalkyle linéaire ou ramifié ayant 1 à 4 atomes de carbone et 1 à 5 atomes d'halogènes identiques ou différents, ou bien
un reste aryle ayant 6 à 10 atomes de carbone ou un reste aralkyle ayant 6 à 10 atomes de carbone dans la partie aryle et 1 à 4 atomes de carbone dans la partie alkyle linéaire ou ramifiée éventuellement substituée par un radical cyano, chacun des deux restes pouvant être substitué dans la partie aryle une à trois fois identiques ou différentes par un halogène, un radical hydroxy, cyano, nitro, un radical alkyle, alkoxy ou alkylthio linéaire ou ramifié ayant chacun 1 à 4 atomes de carbone, un radical cycloalkyle de 3 à 7 atomes de carbone, un radical halogénalkyle, halogénalkoxy ou halogénalkylthio

linéaire ou ramifié ayant dans chaque cas 1 à 4 atomes de carbone et 1 à 5 atomes d'halogènes identiques ou différents et/ou par un groupe phényle qui peut être substitué de son côté une à trois fois identiques ou différentes par un halogène et/ou un radical alkyle ayant 1 à 4 atomes de carbone, ou bien

un groupe hétaryle pentagonal ou hexagonal ou un groupe hétarylalkyle pentagonal ou hexagonal ayant 1 à 6 atomes de carbone dans la partie alkyle linéaire ou ramifiée éventuellement substituée par un radical cyano, la partie hétaryle contenant dans chaque cas 1 à 3 hétéro-atomes tels qu'oxygène, soufre et/ou azote et pouvant être substituée une à trois fois identiques ou différentes par un radical alkyle linéaire ou ramifié ayant 1 à 6 atomes de carbone, halogéno, cyano et/ou halogénalkyle linéaire ou ramifié ayant 1 à 4 atomes de carbone et 1 à 5 atomes d'halogènes identiques ou différents,

$R^7$ est un groupe alkyle linéaire ou ramifié ayant 1 à 6 atomes de carbone, qui peut être substitué une à cinq fois identiques ou différentes par un radical halogéno, cyano, amino, hydroxy, mercapto, nitro, carboxy, des radicaux alkoxy et alkoxycarbonyle linéaires ou ramifiés avec à chaque fois 1 à 6 atomes de carbone dans les parties alkoxy, les radicaux alkylamino et dialkylamino ayant 1 à 6 atomes de carbone dans chaque partie alkyle linéaire ou ramifiée,

ou bien

un groupe alcényle ou un groupe alcynyle ayant chacun 3 à 6 atomes de carbone et substitués chacun le cas échéant par 1 à 5 atomes d'halogènes identiques ou différents, ou bien

un groupe benzyle qui peut être substitué dans la partie phényle une à trois fois identiques ou différentes par un radical halogéno, nitro, cyano, alkyle linéaire ou ramifié ayant 1 à 6 atomes de carbone, de même que par un radical halogénalkyle, halogénalkoxy ou halogénalkylthio linéaire ou ramifié ayant dans chaque cas 1 à 4 atomes de carbone et 1 à 5 atomes d'halogènes identiques ou différents,

$R^8$ représente l'hydrogène ou un groupe alkyle linéaire ou ramifié ayant 1 à 6 atomes de carbone, qui peut être substitué une à cinq fois identiques ou différentes par un radical halogéno, cyano, amino, hydroxy, mercapto, nitro, carboxy, des radicaux alkoxy et alkoxycarbonyle linéaires ou ramifiés ayant dans chaque cas 1 à 6 atomes de carbone dans les parties alkoxy, des radicaux alkylamino et dialkylamino ayant 1 à 6 atomes de carbone dans chaque partie alkyle linéaire ou ramifiée, ou bien

un groupe alcényle ou alcynyle linéaire ou ramifié ayant dans chaque cas 3 à 6 atomes de carbone et chacun étant substitué le cas échéant par 1 à 5 atomes d'halogènes identiques ou différents, ou bien

un groupe benzyle qui peut être substitué dans la partie phényle une à trois fois identiques ou différentes par un radical halogéno, nitro, cyano, un radical alkyle linéaire ou ramifié ayant 1 à 6 atomes de carbone ainsi qu'un radical halogénalkyle, halogénalkoxy ou halogénalkylthio linéaire ou ramifié ayant dans chaque cas 1 à 4 atomes de carbone et 1 à 5 atomes d'halogènes identiques ou différents, ou bien

$R^7$ et $R^8$ forment en outre conjointement un reste alcanediyle divalent ayant 4 à 6 groupes $CH_2$, l'un des groupes $CH_2$ étant éventuellement remplacé par O ou S, pour combattre des micro-organismes indésirables.

**2.** Utilisation suivant la revendication 1, caractérisée en ce qu'on utilise comme dérivé d'acide dibromo-thiophène-carboxylique le composé de formule

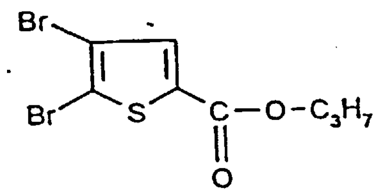

**3.** Utilisation suivant la revendication 1, caractérisée en ce qu'on utilise comme dérivé d'acide dibromo-thiophène-carboxylique le composé de formule

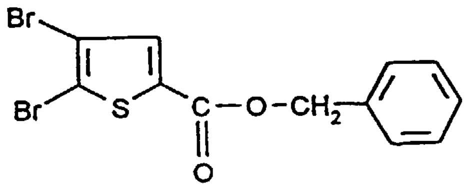

**4.** Dérivés d'acide dibromo-thiophène-carboxylique de formule

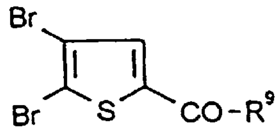

(I-A),

dans laquelle

$R^9$ représente le groupement $-OR^{10}$
où
$R^{10}$ est un groupe alkyle linéaire ou ramifié ayant 1 à 12 atomes de carbone, qui est substitué une à cinq fois identiques ou différentes par un radical halogéno, cyano, amino, hydroxy, mercapto, carboxy, nitro, un radical alkoxy, alkylthio ou alkoxycarbonyle linéaire ou ramifié ayant dans chaque cas 1 à 6 atomes de carbone dans les parties alkyle individuelles,
ou bien
un groupe alcényle de 3 à 6 atomes de carbone, linéaire ou ramifié éventuellement substitué une à cinq fois identiques ou différentes par un halogène,
ou bien
un reste cycloalkyle de 3 à 7 atomes de carbone ou un reste cycloalkylalkyle ayant 3 à 7 atomes de carbone dans la partie cycloalkyle et 1 à 4 atomes de carbone dans la partie alkyle linéaire ou ramifiée éventuellement substituée par un radical cyano, la partie cycloalkyle des deux restes mentionnés pouvant être substituée une à cinq fois identiques ou différentes par un radical alkyle linéaire ou ramifié ayant 1 à 4 atomes de carbone, un halogène, un radical halogénalkyle linéaire ou ramifié ayant 1 à 4 atomes de carbone et 1 à 5 atomes d'halogènes identiques ou différents, un radical cyano, alkylènedioxo ayant 1 à 3 atomes de carbone, un radical cycloalkyle de 3 à 7 atomes de carbone portant éventuellement 1 à 3 substituants, identiques ou différents, alkyle ayant 1 à 4 atomes de carbone, et/ou par un reste phényle qui peut être substitué de son côté une à trois fois identiques ou différentes par un radical alkyle linéaire ou ramifié ayant 1 à 4 atomes de carbone, un halogène et un radical halogénalkyle linéaire ou ramifié ayant 1 à 4 atomes de carbone et 1 à 5 atomes d'halogènes identiques ou différents, ou bien
un reste aryle ayant 6 à 10 atomes de carbone ou un reste aralkyle ayant 6 à 10 atomes de carbone dans la partie aryle et 1 à 4 atomes de carbone dans la partie alkyle linéaire ou ramifiée éventuellement substituée par un radical cyano, chacun des deux restes pouvant être substitué dans la partie aryle une à trois fois identiques ou différentes par un radical halogéno, hydroxy, cyano, nitro, un radical alkyle, alkoxy ou alkylthio linéaire ou ramifié ayant chacun 1 à 4 atomes de carbone, un radical cycloalkyle de 3 à 7 atomes de carbone, un radical halogénalkyle, halogénalkoxy ou halogénalkylthio linéaire ou ramifié ayant chacun 1 à 4 atomes de carbone et 1 à 5 atomes d'halogènes identiques ou différents et/ou par un radical phényle qui peut être substitué de son côté une à trois fois identiques ou différentes par un halogène et/ou par un radical alkyle ayant 1 à 4 atomes de carbone.

**5.** Procédé de production de dérivés d'acide dibromo-thiophène-carboxylique de formule (I-A) suivant la revendication 4, caractérisé en ce qu'on fait réagir des halogénures d' acide dibromo-thiophène-carboxylique de formule

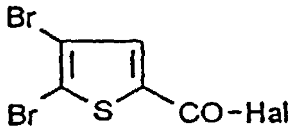

(II),

dans laquelle

Hal représente un halogène, avec des alcools de formule

$$HO\text{-}R^{10} \text{ (IV)} \qquad \text{(IV)}$$

dans laquelle

$R^{10}$ a les définitions indiquées ci-dessus,

éventuellement en présence d'un diluant et en la présence éventuelle d'un auxiliaire de réaction, de même que, le cas échéant, en présence d'un catalyseur.

**6.** Compositions microbicides, caractérisées par une teneur en au moins un dérivé d'acide dibromo-thiophène-carboxylique de formule (I) suivant la revendication 4, à côté de diluants et/ou d'agents tensio-actifs.